# EUROPEAN PATENT SPECIFICATION

(11) **EP 4 486 237 B1**
(45) Date of publication and mention of the grant of the patent: **01.04.2026**
(21) Application number: 23709370.3
(22) Date of filing: 02.03.2023
(51) Int. Cl.: A61B 18/02, A61F 7/00

(54) **ELECTRICALLY OPERATED CRYOCABIN ARRANGEMENT**
ELEKTRISCH BETRIEBENE KRYOKABINENANORDNUNG
AGENCEMENT DE CRYOCABINE À COMMANDE ÉLECTRIQUE

(30) Priority: 03.03.2022 FI 20225191
(43) Date of publication of application: 08.01.2025
(73) Proprietor: Cryotech Nordic OÜ, 13525 Tallinn, Harju maakond (EE)
(72) Inventor: YLIOLLITERVO, Juha, 04430 Järvenpää (FI); MARTINS, Johanna, 02920 Espoo (FI)
(74) Representative: Heinonen & Co
(86) International application number: PCT/EP2023/055302
(87) International publication number: WO 2023/166121

(56) References cited:
- EP-A1- 3 628 280
- WO-A1-2020/260347
- CN-A- 109 157 323
- US-A1- 2017 209 302

## Description

The present invention generally relates to devices for whole-body cryotherapy. In particular, the invention concerns an electrically operated whole-body cryocabin arrangement with improved cooling uniformity, and a computer program product.

### BACKGROUND

Whole-body cryotherapy or whole-body cold therapy (WBCT) is a recognized procedure for promoting muscle recovery and for enhancing general wellbeing of an individual. Upon impact of air-infused cryogenic liquids, whole-body cryotherapy treatment is believed to invoke and/or reactivate internal resources of the organism, thus promoting its natural capacity for a self-defense against various diseases including asthma, hormone deficiencies, joint inflammation and skin disorders, such as allergies and psoriasis. Long-term effects of cryotherapy include an enhanced immune resistance of the organism and an improved flexibility and elasticity of soft tissues and skin. Cold treatments also promote cell replacement processes naturally occurring within the body, and elimination of dead cells, accordingly.

Cryotherapy treatment aims at non-invasively applying thermal (cold) stimuli over a body area of a patient to induce an intense sensory response (a so-called thermal shock response, in present case - cold shock response) in skin and an underlying (soft) tissue thereupon the aforementioned (cryo)therapeutic effect(s) take place. Sensory response of a desired/required intensity is induced upon a sudden cold impact over a plurality of body areas, whereby the body develops local stress responses, followed by activation of metabolic processes, acceleration and intensification of blood circulation, increased oxygen supply to blood and tissues and release of anti-inflammatory and analgesic substances, that altogether results in at least alleviation of pain, reduced swelling and diminished muscle tension.

The required cold shock response can be achieved by a variety of methods. In conventional WBCT chambers operated by cryogenic coolants, such as (liquefied) carbon dioxide (CO₂) and liquid nitrogen (LN₂), the patient is exposed to an impact of extreme temperatures, such as below -100 degree Celsius, typically within a range of - 110 °C to -170 °C, for short time spans, typically 0,5-3 minutes at a time.

As an alternative to cryogenic liquid operable WBCT chambers, electric cryotherapy chamber solutions are available on the market. In electric cryochambers, temperature reduction is achieved without cryogenic liquid, but through the use of gaseous medium, typically air, cooled to below -100 °C by electrically powered cooling aggregate. However, cooling power of a majority of such devices is too low for generating a cold-induced sensory response of a required intensity, i.e. intensity sufficient for achieving the (cryo)therapeutic effect(s). On the other hand, those solutions that do produce temperatures below -100 °C are extremely expensive. Typical electric-powered cryochamber is a closed-space chamber (not open-top) with essentially no air circulation in its' interior. In an absence of air circulation, heat exchange between the patients' skin and the ambient is markedly reduced.

One common problem associated with the devices for whole-body cryotherapy known from the art (both cryogenic liquid- and electric operable) is poor adjustability of treatment parameters, such as temperature, velocity and/or direction of coolant flow, throughout the cryotherapy session. An exemplary solution disclosed in the U.S. patent No. 4,838,270 (Donnerhack et al) teaches adjusting the directions of cold gas flowing into the cryotherapy cabin before the treatment. Mentioned patent concerns a walk-in, open-top cabin in the form of a half-shell, in which cold gas produced by mixing dry air with liquefied nitrogen can be directed into the cabin via nozzles rotatable about their vertical axis in accordance to height and body shape of the patient.

Known cryoliquid- or electric cooling based solutions do not usually take an account of the fact that ability to withstand cold largely varies amongst individuals. For example, intensity of blood flow circulation in skin and underlying tissue, naturally varying amongst individuals, has a significant impact on temperature conditions at which a strong thermal sensory response is attained at skin surface. Some individuals could not fully benefit from cryotherapy treatments due to personal cold intolerance. Additionally, some body regions are generally more cold-sensitive than the others (local sensitivity). Therefore, during a standard whole-body cryotherapy session, such patients are subjected to the risk of cold injury. Nonetheless, shortened or conducted at higher temperatures treatment session results in reduced treatment efficiency, since conditions required for achieving the sufficiently intense thermal sensory response on skin become more difficult to reach.

It is evident that in an absence of a thermal sensory response of desired/required intensity (a so-called thermal shock response), the treatment cannot be considered effective.

Adjusting flow speed variables in response to body temperature measured from the skin of a user in the whole-body cryocabin arrangement has been recognized in WO 2020/260347 A1 (Martins, Yliollitervo). Disclosed cryocabin arrangement comprises a separate cooling unit and fluid (re)circulation units. The document further teaches that a thermal sensory response of required intensity could be achieved at threshold temperatures within a range of -1 to 0 °C.

However, there is still room for improvement in technologies related to equipment and methods used in whole-body cold treatments, in particular, related to developing improved concepts for controlling coolant circulation- and/or temperature related parameters inside a cold treatment chamber in a user-specific manner to create customized treatment experiences.

### SUMMARY OF THE INVENTION

An objective of the present invention is to solve or at least alleviate each of the problems arising from the limitations and disadvantages of the related art. The objective is achieved by various embodiments of an electrically operated whole-body cryocabin arrangement, a computer-implemented method for operating the same and a computer product adapted to perform the method, according to what is defined in the independent claims.

In an aspect, an electrically operated cryocabin arrangement is provided, according to what is defined in the independent claim 1 and a computer program product to operate the cryocabin is disclosed in independent claim 10.

In embodiment, the electrically operated cryocabin arrangement is provided, comprising an open-top cabin with a number of thermal sensor arrays arranged at different heights along an internal perimeter of the cabin, a number of cooling units, and a data processing unit, wherein the data processing unit is configured to receive user-specific data comprising at least temperature indications (*t1*) (non-invasively) measureable at skin surface of a user by the thermal sensor arrays throughout an operation cycle, during which cycle a cooling fluid is delivered into the cabin and distributed inside the cabin via the cooling units, and based on said user-specific data, to selectively adjust variables related to distribution of the cooling fluid inside the cabin to a predetermined level, said variables being at least a flow velocity (V) of a cooling fluid stream and/or a temperature (*t2*) of the cooling fluid; wherein the cooling fluid distribution variables are adjusted to reach the values, at which temperature indications (*t1*) measureable at the user skin surface are within a range of about 0 degree Celsius (°C) to about 20 °C, and wherein adjustment is performed in real time, continuously throughout the operation cycle.

In embodiment, in the cryocabin arrangement, the temperature (*t2*) of the cooling fluid distributed inside the cabin is adjusted to a temperature value equal to or above about -60 °C, preferably, to the temperature value within a range of between about -60 °C to about -15 °C.

In embodiment, the flow velocity of the cooling fluid stream distributed inside the cabin is adjusted to a range of between about 1-15 m/s. In embodiment, the cooling fluid distributed inside the cabin is air.

In embodiment, in said cryocabin arrangement, the processing unit is configured to terminate the operating cycle automatically when a desired temperature value (*t1*) measureable at the user skin surface has been achieved.

In embodiment, the data processing unit is further configured to combine the user-specific temperature measurement data (*t1)* obtainable continuously throughout the operation cycle with a supplementary user-specific data selected from the group consisting of: height, weight, age, and gender, and inputted into the processing unit before the beginning of the operating cycle.

In embodiment, the cooling unit comprises a heat exchanger device and at least one blower device configured to deliver, distribute, and preferably recirculate the cooling fluid inside the cabin. In embodiment, the heat exchanger device is a heat exchanger evaporator.

In embodiment, in said cryocabin arrangement, recirculation of the cooling fluid is enabled between the cooling units and the cabin in a substantially closed-loop cycle.

In embodiment, the thermal sensor arrays are infrared sensor arrays, such as thermopiles.

In embodiment, the cabin of the cryocabin arrangement further comprises a removable top cover with a neck collar portion.

In embodiment, the computer implemented method is provided for operating a cryocabin arrangement comprising an open-top cabin with a number of thermal sensor arrays arranged at different heights along an internal perimeter of the cabin, a number of cooling units, and a data processing unit, the method comprises at least the following steps:
(a) receiving, into the data processing unit, user-specific data comprising at least temperature indications (*t1*) non-invasively measured from skin surface of a user by the thermal sensor arrays in a contactless manner;
(b) processing the user-specific measurement data in the data processing unit, and
(c) based on processed user-specific measurement data, generating, in the data processing unit, an output signal or a series of output signals configured, when transmitted to the cooling units, to selectively adjust variables related to distribution of a cooling fluid inside the cabin to a predetermined level, said variables being at least a flow velocity (*V*) of a cooling fluid stream and/or a temperature (*t2*) of the cooling fluid, wherein the output signal(s) generated by the data processing unit (30) is/are configured to selectively adjust cooling fluid distribution variables to reach the values, at which temperature indications (*t1*) measureable at the user skin surface are within a range of about 0 degree Celsius (°C) to about 20 °C, and
wherein the method steps (a), (b) and (c) are performed in real time throughout an entire operation cycle, during which cycle the cooling fluid is delivered into the cabin via the cooling unit and recirculated inside said cabin by fluid circulation units.

Utility of the present invention arises from a variety of reasons depending on each particular embodiment thereof. Overall, the invention enables a cryocabin arrangement, in which, during an operation cycle, a variety of cabin-related (or flow-related) parameters can be monitored and adjusted with high precision and taking into account personal sensitivity to cold. Said parameters include, but are not limited to source, temperature, velocity (speed rendered with a direction vector), speed and direction of fluidic stream(s) distributed inside the cabin. By enabling monitoring and personalized adjustment of coolant flow related parameters in real time, the invention allows for generating fluidic streams entering the cabin at predetermined temperature and flow speed to target selected body areas in a highly customized manner. Thus, by individually adjusting fluid flow directed to particular body areas (within the same cabin) naturally having different perception to cold, it is possible to achieve the sensory response of essentially the same intensity (uniform cold shock response) essentially simultaneously in all treated body area (viz. the entire body). Hence, cooling uniformity achievable during the operation cycle is improved and excessive cooling of body areas with particular sensitivity to cold is avoided. At the same time, the body areas less sensitive to cold could still receive adequate treatment sufficient to attain the thermal sensory responses of required intensity, i.e. intensity sufficient to induce health and/or wellbeing promoting effects.

Thermal sensitivity varies approximately 100-fold over the body surface. Therefore, an arrangement, by which the cold-induced thermal sensory response of required intensity could be attained uniformly throughout the entire body of an individual (that is, at the body areas having different sensitivity to cold), is undoubtedly beneficial, in terms of both treatment efficiency and cost-effectiveness, for a person whose intention is to undergo a series of treatments by cryotherapy

In the cryocabin arrangement disclosed hereby, the cold-induced thermal sensory response of required intensity can be attained uniformly throughout the entire body of an individual by monitoring and adjusting, in a real-time regime, delivery of cooling fluid to particular body areas. It has been observed that with the proposed device and operating method, the uniformity of delivery of said cooling fluid to particular body areas (viz. cooling uniformity) has been further improved. Adjusting the body temperature of a user to a predetermined level in a highly uniform manner, wherein all body areas are cooled to temperatures needed for achieving the cold shock response essentially simultaneously, is achieved by selective regulation of flow-related parameters during an operation cycle.

The invention is preferably realized as an electrically cooled cabin solution, which does not require utilization of cryogenic coolants (liquid gases). Restorative and health-promoting effect(s) stipulated by generation of controlled thermal sensory response are achieved for the entire body at temperatures much higher than that utilized for conventional cryotherapy. In fact, the invention allows for attaining health and wellbeing promoting effects typically associated with invoking the so-called thermal shock response by adjusting the temperatures at the user skin surface to the values above zero (degree Celsius).

The invention further allows for adjusting parameters related to cooling fluid distribution inside the cabin such, as to establish customized treatment conditions suitable for users with low-, medium- and high perception threshold for cold.

The term "a user" is utilized in present disclosure to indicate a subject, such as a human or a nonhuman mammal, positioned inside the cryocabin.

The terms "speed" and "velocity" are generally used interchangeably; however, a reference is made specifically to "velocity" to emphasize that the speed has direction.

The expression "a number of" refers in present disclosure to any positive integer starting from one (1), e.g. to one, two, or three. The expression "a plurality of" refers to any positive integer starting from two (2), e.g. to two, three, or four.

Different embodiments of the present invention will become apparent by consideration of the detailed description and accompanying drawings.

### BRIEF DESCRIPTION OF THE DRAWINGS

Fig. 1 is a perspective view of a cryocabin arrangement 100 according to an embodiment.
Fig. 2 illustrates a vertical crosscut of the cryocabin arrangement, according to the embodiment, and shows a user accommodated in the cabin.
Fig. 3 illustrates a horizontal crosscut (top view) of the cryocabin arrangement according to the embodiment.
Fig. 4 is a flowchart of a computer implemented method to operate the cryocabin arrangement.

### DETAILED DESCRIPTION OF THE EMBODIMENTS

Detailed embodiments of the present invention are disclosed herein with the reference to accompanying drawings. The same reference numerals are used throughout the drawings to refer to same members as follows:
100 - a cryocabin arrangement;
101 - a user;
10 - a cabin;
10A - an interior of the cabin;
10B - insulation;
11 - an elevator plate;
13 - sensor array(s);
14 - a removable top cover;
15 - a monitor display;
16 - ventilation grid(s);
19 - a compressor unit;
20 - a cooling unit;
21 - heat exchangers;
22 - auxiliary fluid distribution devices;
23 - a blower device;
30 - a processing unit;
31 - a control terminal;
201 - streams of cooling fluid delivered into the cabin from the cooling unit 20 and recirculated inside the cabin.

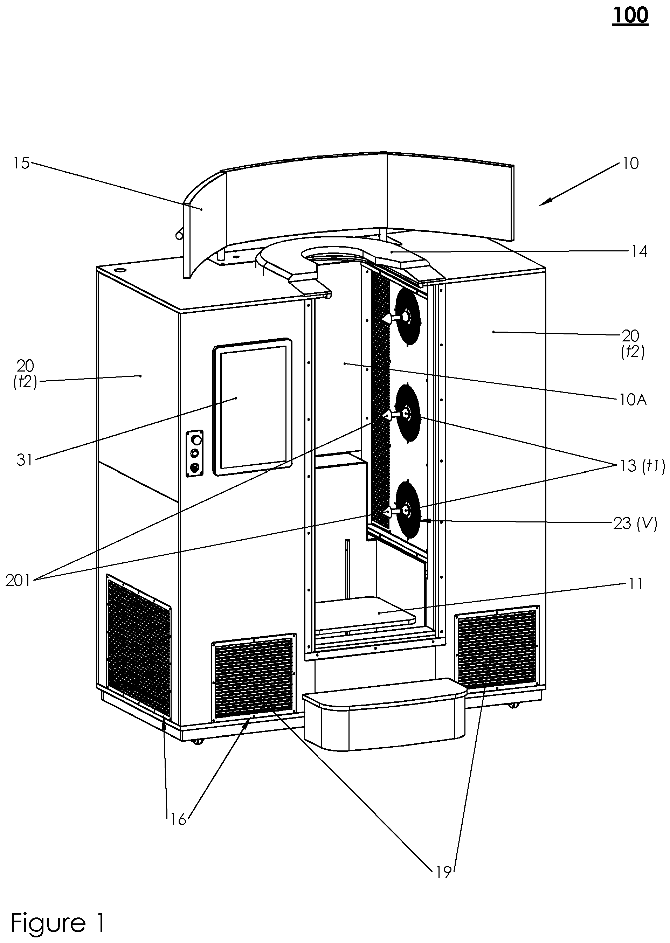

Figs. 1-3 illustrate a cryocabin arrangement 100 according to the embodiments.

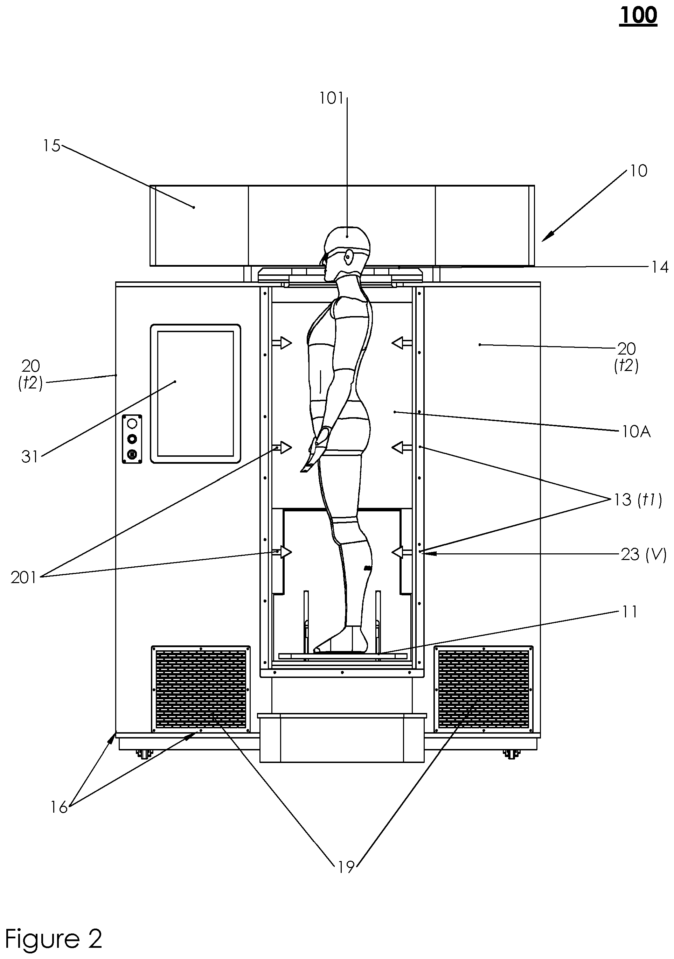

The arrangement 100 comprises a cabin 10 and a number of cooling- and fluid (re)circulation units 20, hereafter, the cooling units 20. The cabin 10 is generally configured as an open-top chamber with an interior 10A suitable for accommodating a user. In presented configurations, the cabin 10 is configured as a vertical open-top chamber suitable for accommodating a standing adult human in a manner shown on Fig. 2.

It should be noted that upon appropriate modifications, the cabin 10 can accommodate a standing nonhuman mammal. In particular, the cabin can be modified to accommodate a so-called companion animal or a pet, such as a canine, an equine, and any other nonhuman animal species.

The cabin comprises a base and a door (not shown); the door can be a sliding door or a hinged door. The base is preferably equipped with an elevator plate or a platform 11 configured to move up and down along a vertical axis of the cabin, so that the cabin interior can be adapted to the height of the user. By means of the elevator plate 11, position of the user in the cabin is adjusted such that the upper edge of the cabin is approximately at a neck level of the user, whereby head remains above the open-top upper edge (outside of the cabin).

In some configurations, the arrangement 100 further comprises a removable top cover 14 with a neck collar portion. The top cover 14 is placed on the top of the cabin 10 (otherwise open) to create an essentially contained volume and to prevent cooling fluids from uncontrollably leaving the cabin. The neck collar portion of the top cover is designed to span around the user's neck in a non-containing manner. The neck collar portion may thus comprise a slit, it may be composed of two halves, for example, or it may follow a tubescarf design. An opening provided in the neck collar portion can be made adjustable to encircle the neck of a user of any size.

The cabin may optionally comprise a number of illumination devices configured as spotlight sources located along the upper edge of the cabin and/or one or more illumination devices above the cabin (not shown). Location and number of illumination devices may vary depending on a particular cabin design.

A monitor display or displays 15 (Figs. 1, 2) may be arranged above the cabin. The monitor display(s) may be used for demonstrating any one of: treatment related parameters, such as time, ambient temperature, pressure, humidity, and the like; user-related parameters, such as for example real-time readouts from thermographic camera(s); and/or any kind of entertainment media.

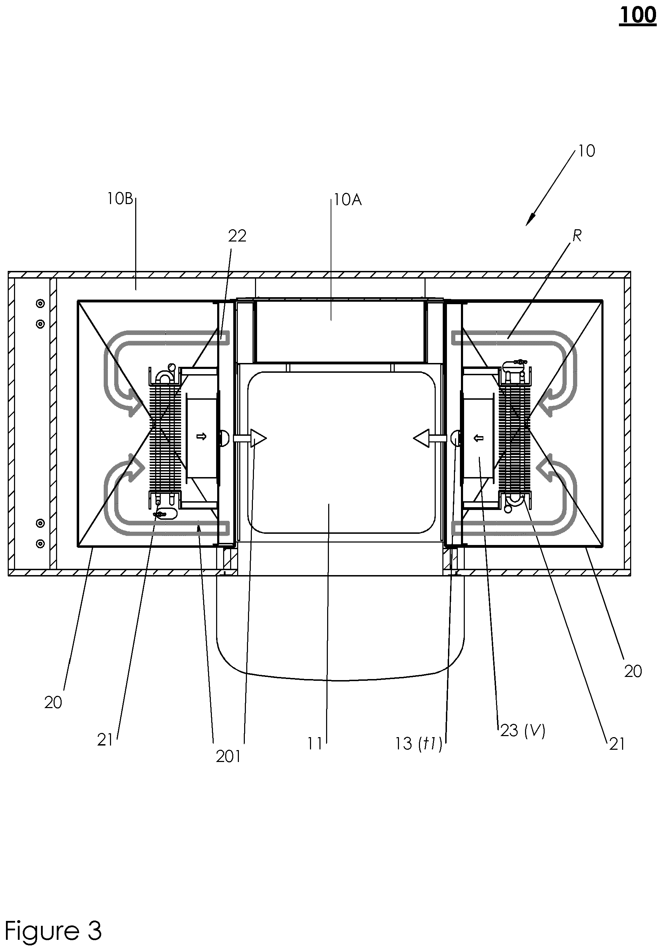

The cabin 10 is thermally insulated. (rf. Fig. 3, 10B). In some instances, a number of (optional) protection shields/screens is placed against the cooling units 20 to protect the user from direct cold streams (not shown). The interior of the cabin 10 including protection shields may be padded by a suitable cold-resisting material. The shields can be configured as temporary, easily dismountable appliances.

Each of the cooling units 20 can be configured as a vertically standing unit. The cooling unit comprises a heat exchanger device 21 and at least one blower device 23. Each cooling unit may incorporate 2-12 blower devices, for example. The blower device can be a fan or an impeller, for example. In configurations, the heat exchanger device is a heat exchanger evaporator.

The cooling unit is configured to deliver, distribute, and preferably recirculate a cooling fluid inside the cabin. The cooling fluid, delivered into the cabin and further distributed / (re)circulated inside the cabin via the cooling- and fluid (re)circulation units 20, is designated by the reference number 201.

Fluidic medium used for cooling is preferably a gaseous medium, such as air (ambient air), which is pressurized and optionally pre-cooled in a compressor unit or units 19 (rf. Figs. 1 and 2), for example, and further guided into the cooling unit(s) 20.

In the cryocabin arrangement 100, recirculation of the cooling fluid is enabled between the cooling units 20 and the interior of the cabin 10, 10A in a substantially closed-loop cycle. The closed-loop recirculation of the cooling fluid between the cooling units and the cabin is indicated on Fig. 3 with arrows *R*. The cooling units 20 are thus configured to intake the cooling fluid delivered into to cabin and to further return (re)circulated cooling fluid back into the cabin. In the cooling unit 20, the cooling fluid is (re)circulated through the heat exchanger 21, such as the heat exchanger evaporator, and the blower device(s) 23. Via the blower device(s) 23, the cooling fluid is directed into the cabin.

Intake of the cooling fluid into the cooling units 20 from the cabin 10 may be enabled by a number of auxiliary fluid distribution devices 22 (rf. Fig. 3). The latter may comprise any types of nozzles or spouts suitable for directing fluids in a predetermined direction. The auxiliary fluid distribution devices 22 may comprise suction nozzle(s) for intake of fluids and optionally any suitable blowing nozzle(s) for return of fluids. In some configurations, the blower devices 23 and the auxiliary fluid distribution devices 22 are arranged essentially along the entire height of the cooling unit(s) and essentially along the entire height of the cabin, respectively, in order to target all body regions, such as the upper body / chest region, waist region, the lower body and the legs.

In some instances, the auxiliary fluid distribution devices may be configured to enable regulated fluid flow in multiple directions. For example, these auxiliary devices may direct fluids along a path inclined at an angle within a range of 30-45-50 degree, related to vertical walls of the cabin, in any one of upward and downward directions. Some nozzles can be configured as automatically adjustable rotating nozzles (e.g. ball-type nozzles), that enable adjusting orientation / jet direction of the nozzle within a range of 0-50 degree.

In some configurations, the cabin may be provided only with suction nozzles for intake of the cooling fluid into the cooling units 20. Return of the cooling fluid may be realized directly via the blower devices 23. In such as event, the blower devices are arranged behind protective screens configured to enable undisturbed fluid flow into the cabin 10, 10A.

In presented configurations, the arrangement 100 comprises two cooling units 20 arranged at both sides of the cabin essentially opposite one another (Figs. 1-3). Provision of the cryocabin arrangement with at least one cooling unit is possible.

The cryocabin arrangement 100 is preferably electrically operated. The arrangement thus comprises at least one electric motor (not shown) positioned such that heat produced thereby does not affect the cabin 10, or any one of the cooling units 20. The blower devices 23 can be driven by electric power produced by the electric motor.

Ventilation grids are designated by a reference numeral 16.

Flow velocity of stream(s) of the cooling fluid 201 distributed inside the cabin through the cooling units 20 is dynamically adjustable (described in detail herein below). In practice, the flow velocity of the cooling fluid stream(s) distributed inside the cabin 10 is adjusted to a range of between about 1-15 meter per second (m/s). In some exemplary configurations, the flow velocity is adjusted to about 5-9 m/s.

Also the stream(s) of cooling fluid 201 distributed inside the cabin 10 are dynamically adjustable to predetermined temperature values *t2*. Adjustment of the temperature *t2* can be realized via the heat exchangers 21. The value *t2* can be equal to or exceed about -60 degree Celsius (°C). Sign "-" preceding a numerical indication (such as -60 °C, for example) stands for "minus", i.e. indicates a temperature below zero degrees Celsius. In embodiments, temperature of the cooling fluid 201 is maintained within a range of about -60 °C to about -15 °C. In some exemplary configurations, the temperature range may be maintained at any value within a range of about -35 °C to about -20 °C.

A combination of speed or velocity (*V*) and temperature (*t2*) values at which the cooling fluid 201 is directed towards the user positioned in the cabin 10 is selected such, as to achieve a required cold-induced thermal sensory response (also known as thermal shock response) at a skin surface and the underlying tissue in a most straightforward and efficient manner.

In a non-limiting example of an operating cycle, the cooling unit 20 is configured to produce fluidic streams 201 that enter the cabin and are further distributed and recirculated inside the cabin at a speed of about 1-15 m/s. The fluidic flow having the predetermined speed or velocity (*V*) is generated by the blower devices 23. In some instances, the speed of the cooling fluid flow is adjusted to about 5-9 m/s, while the temperature (*t2*) of the cooling fluid is maintained within a range of about - 20 °C to about -35 °C. Temperature of the cooling fluid flow is preferably adjusted by the heat exchanger device(s) 21.

Any one of the heat exchanger(s) 21, the blower device(s) 23 and the auxiliary fluid distribution devices 22, such as (intake) nozzles, can be controlled locally by means of internal controllers (not shown), such as for examples relays, valves (e.g. solenoid valves), related actuators and other devices. Through these controllers, flow velocity (viz. speed of fluidic stream following a predetermined direction, *V*) and temperature (*t2*) of the cooling fluid stream distributed inside the cabin can be regulated based on an input received into a central processing unit 30, as described further below.

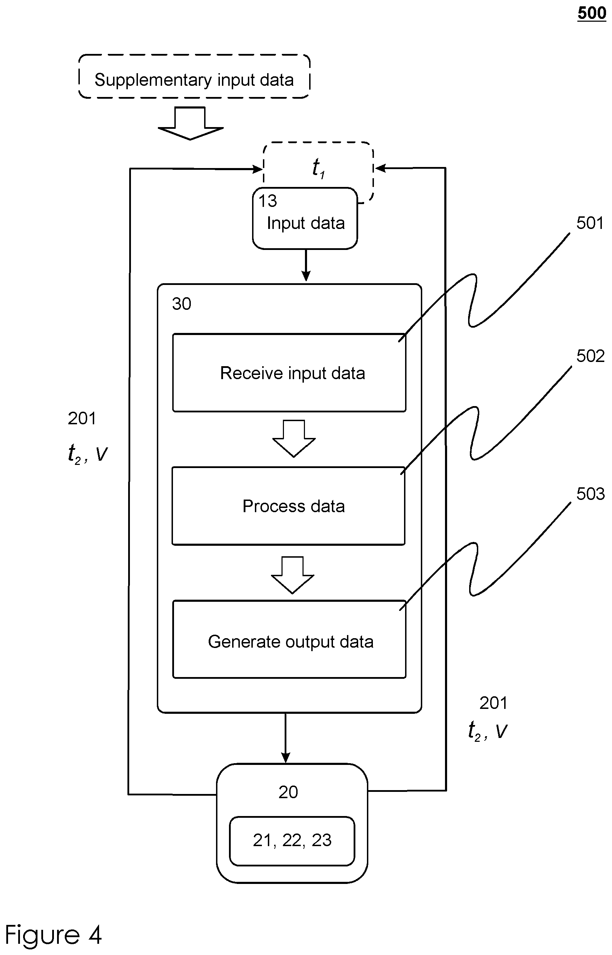

The cabin arrangement 100 further comprises a plurality of sensor devices arranged into a number of sensor arrays 13 disposed in the interior 10A of the cabin. The sensor array or arrays 13 are disposed at different heights along the internal perimeter of the cabin (on internal walls and, optionally, on an inner side of the door) to obtain measurements uniformly across the entire interior 10A of the cabin. The sensor devices from the plurality of the sensor devices forming the sensor arrays 13 are preferably configured as thermal imaging sensors / transmitters, that measure temperature (*t1*) at skin surface of the user and optionally the temperature (*t2*) in the cabin throughout the operation cycle and transmit measurement data to the processing unit 30 (Fig. 4). The cabin can be further provided with a group of other sensor/detector devices configured to measure some additional variables, such as pressure, humidity, presence of chemical substances in the ambient, and the like.

During the operating cycle, the sensor devices (sensor arrays 13) located on internal walls of the cabin measure the temperature (*t1*) at the skin surface of the user non-invasively, in a contactless manner.

Thermal imaging sensor arrays 13 that measure temperature (*t1*) at skin surface of the user preferably comprise a plurality of infrared thermal imaging sensors. Still, any other sensor devices capable of measuring temperature and/or detecting heat emitted from skin surface of the user positioned in the cabin 10 can be utilized.

The sensor arrays 13 may be configured as thermal imaging (thermographic) cameras integrated in the interior of the cabin 10. Altogether, such cameras generate a high-resolution thermal map based on temperature variables *t1* measured at the user skin surface.

User-specific measurement data obtainable by the sensor arrays 13 during the operating cycle form an essential part of the input data received by the processing unit 30. In the processing unit, the measurement data are analyzed, and an output is generated in the form of signals transmitted to the cooling units 20 for adjusting a number of selected variables related to distribution of the cooling fluid 201 inside the cabin to a predetermined level. Separate signals can be transmitted to the heat exchanger(s) 21 and to the blowers 23, via related controllers. Adjustable variables include at least a flow velocity of a cooling fluid stream and/or a temperature of the cooling fluid flow conveyed into the cabin 10 from the cooling units 20.

In some instances, supplementary user-specific data can be entered into the proceeding unit 30 (via the control terminal 31) prior to beginning of the operating cycle. Such data comprises information about the user, such as height, weight, age, gender, and the like. During data processing, the supplementary user-specific data can be optionally combined with the measurement data obtained from the sensor arrays 13.

Obtaining measurement (input) data from the sensor arrays 13, processing said data, and producing an output (a series of commands to render fluidic stream(s) 201 with a predetermined temperature (*t2*) and/or velocity (*V*) and optionally selecting a blower device 23 from a number of blower devices) is conducted in real time continuously during the operation cycle. Cooling fluid related parameters (temperature and speed / velocity) are typically adjusted via the cooling unit 20 and associated controls (e.g. temperature, pressure, fan rotation speed, etc.).

The (central) processing unit 30 can be integrated within the cabin arrangement 100 or provided as a standalone solution, e.g. as a local- or remote computer system, for example, including, but not limited to PC, portable or tablet computer, a mobile device such as a smartphone or a tablet, and the like. The computer system may be further configured to display measurement parameters obtainable from the sensor arrays 13, for example, in the form of visualizations (e.g. thermographic images / thermal maps in 2D and/or 3D). These visualizations, generally available to an operator of the cryocabin arrangement 100, can be further synchronized with images visible to the user via the displays(s) 15, for example. The computer system may, for example, present temperature variables (*t1, t2*) measurable with regard to different body regions during the operating cycle as a three-dimensional interactive visualization.

The cabin arrangement further comprises a control terminal 31 (Figs. 1, 2) with a user interface, such as a graphical user interface. It can be realized in the form of a display screen, such as a touchscreen, for example. User interface may also comprise an at least one audio input-output device and an associated circuitry. Figs. 1, 2 show provision of the control terminal 31 integrated into the cabin 10, such as into a sidewall of the cabin. The control terminal 31 is connected to the processing unit 30 in wired and/or wireless manner, i.e. through wired infrastructure and/or through a wireless network.

The processing unit 30 may comprise one or more data processing devices containing a processing circuitry capable of interpreting and executing instructions input via the user interface, said processing devices being realized as microprocessors, microcontrollers, digital signal processors, programmable logic chips etc.

The processing unit 30 and the control terminal 31 are in data communication with each of the sensor arrays 13 and the controllers regulating delivery and distribution of the cooling fluid 201 into the cabin 10 through the cooling units 20.

In some configurations, the cabin arrangement 100 further comprises a number of vaporizer devices (not shown) configured to deliver into the cabin an aqueous-based solution prior to or during the operation cycle. The vaporizer devices are preferably configured as spray vaporizers, jet-vaporizers, or atomizers. The solution is supplied into the cabin from a number of replaceable containers (not shown) disposed outside the cabin. Spraying the aqueous-based solution (a mixture of water, optionally micellar water with oils, for example) may be useful in promoting heat extraction from skin during the operating cycle. For example, it has been observed that using micellar aqueous solutions combined with vegetable oils increase heat extraction from skin surface almost twice, as compared to plain water. However, delivery of said solution into the cabin is not necessary to achieve the desired thermal sensory effect.

In some instances, the cabin arrangement may comprise a number of spot-light devices optionally arranged into arrays (not shown) installed on internal walls of the cabin and configured to emit light within a predetermined portion of the electromagnetic spectrum, preferably, within the visible spectrum, defined as the wavelength range of electromagnetic radiation between 380 and 750 nanometers (nm). In some instances, each device 13 can be configured to emit light at a single fixed wavelength, such as blue (about 490 nm to about 450 nm), green (about 560 nm to about 520 nm) and/or red (about 620 nm to about 750 nm). In such an event, the illumination devices 13 can be advantageously provided as Light Emitting Devices (LEDs) or any other devices appropriate for use, in particular, in view of development of the technology.

In some instances, combining light sources configured to emit light at different wavelengths into arrays is advantageous due to ability of light emitted at various wavelengths to target particular dermal conditions. Thus, blue light is known to effectively treat a variety of dermal infections, such as acne and related skin inflammations. Green light is, in turn, known to targets dark circles, pigmentation, and the like. Additionally, green light calms irritated skin and it is often used in the anti-age treatments. Red light accelerates blood circulation at skin surface and increases collagen production, which is essential for various skin stimulation- and rejuvenation (cosmetic) treatments.

Overall, the cryocabin arrangement 100 can also be used in non-therapeutic treatments, for example in non-therapeutic (cosmetic) treatments of skin conditions, such as acne, cellulitis etc.

Reference is made to Fig. 4 illustrating a method 500 for operating the cryocabin arrangement 100. In embodiments, the method 500 is a computer implemented method. The cabin arrangement 100 is advantageously configured to implement said method.

In the method, the user-specific data is received, at 501, into the processing unit 30. The user-specific data received at 501 comprises at least temperature indications (*t1*) non-invasively measured from skin surface of a user in a contactless manner by the sensor arrays 13. The user-specific data may contain information on heat extraction detected and/or measured at the skin surface of the user positioned into the cabin 10.

At 502, the user-specific data is analyzed in the processing unit 30.

The method continues at 503, wherein, based on said user-specific measurement data, an output signal or a series of output signals is/are generated in the processing unit 30 and transmitted to the cooling units 20. Based on these output signal(s), the cooling units 20 selectively adjust a number of variables related to distribution of the cooling fluid 201 inside the cabin to a predetermined level. Adjustable variables include at least a flow velocity (*V*) of a cooling fluid stream (adjustable by the blower devices 23) and/or a temperature (*t*2) of the cooling fluid (adjustable by the heat exchangers 21). A predetermined number of the blower devices 23 can be further selected for directing stream(s) 201 into the cabin.

In the method, the output signal or a series of output signals generated, at step 503, by the data processing unit 30 is/are thus configured, when transmitted to the cooling units, to selectively adjust variables related to distribution of the cooling fluid 201 inside the cabin to a predetermined level.

In embodiment, the method comprises adjusting distribution the cooling fluid 201 (fluid flow variables *V, t*2) (re)circulated inside the cabin through the cooling- and fluid circulation units 20.

Steps 501-503 are performed in real time throughout an entire operation cycle, during which cycle the cooling fluid is delivered into the cabin and distributed and preferably recirculated inside said cabin by the cooling units 20.

The data received from the thermal sensor arrays 13 at step 501 concerns the temperature indications measurable at the user skin surface (variable *t1*). In preferred embodiments, the sensor arrays 13 are configured to detect a particular region or regions on skin surface at which the temperature has reached or about to reach the values within a range of about 0 °C to about 20 °C, to register and to transmit these indications to the processing unit 30. In the method, the output signal or a series of output signals generated by the data processing unit 30 is/are configured to selectively adjust the cooling fluid distribution variables to reach the values, at which temperature indications (*t1*) measureable at the user skin surface are within a range of about 0 °C to about 20 °C. In some configurations, the cooling fluid distribution variables are adjusted to reach the values, at which temperature indications (*t1*) measureable at the user skin surface exceed zero degree Celsius (> 0 °C), preferably falling into a range of about 1 °C to about 20 °C.

By adjusting fluidic flow related distribution parameters during the operation cycle, as disclosed hereinabove, a controlled cold impact can be applied onto the entire body of the user (excluding head), whereby the temperature at skin surface across the entire body area can be decreased to reach the temperature indications (*t1*) within a range of about 0 °C to about 20 °C. By regulating parameters related to distribution of the cooling fluid 201 inside the cabin, such as at least the flow velocity of the cooling fluid stream (*V*) and/or the temperature of the cooling fluid (t2), based on the data obtained by the thermal sensor arrays 13, the user-specific temperature indications *t1* can reach desired values (0-1 °C to 20 °C) throughout the entire body of the user in a highly uniform manner. All body areas (including the body areas having high-, moderate- and low perception threshold for cold impact) can be cooled essentially simultaneously.

During the operating cycle, fluid distribution and (re)circulation related variables (*V, t*2) are adjusted to and maintained at a level sufficient to reach and maintain the temperature variable(s) *t1* measureable at the user skin surface, within a range of about 0 °C to about 20 °C. Duration of each operating cycle is determined individually.

By way of example, typical duration of the operation cycle is about 1-4 min. It has been experimentally determined that this period is usually sufficient to achieve the thermal sensory response of required intensity at skin surface and underlying tissue of the user.

The processing unit 30 of the cryocabin arrangement is preferably configured to terminate the operating cycle automatically when the desired temperature value (*t1*) has been achieved at the skin surface. That the body area has been cooled to a predetermined, desired temperature *t1* and that the desired temperature value has been achieved is determined based on readouts obtained from the sensor arrays 13. In an event the sensor readouts are stable (show essentially the same value) during a certain period of time (e.g. 30 sec to 1 min), and the fluid flow related parameters (*t*2, *V*) need not be adjusted, accordingly, the temperature *t1* is considered achieved and the operating cycle is terminated respectively. A stability determination threshold (a predetermined period of time) can be entered into the processing unit 30 beforehand. The threshold may be same or different over the treated body areas or groups of body areas. In such a way, efficient treatment of the body areas having different sensitivity to cold can be realized.

During experimental trials underlying the present invention, it has been observed that at a predetermined combination of parameters related to cooling fluid distribution inside the cabin (*V, t*2), adjusting the temperature variable *t1* at skin surface to a level essentially exceeding zero degree Celsius (> 0 °C), is sufficient to develop the cold-induced thermal sensory response of required intensity in skin and the underlying tissue, such as subcutaneous tissue.

It has been observed that the temperature variable *t1* measurable at the skin surface reaches the values between about 0 °C to about +20 °C, when the temperature regime *t*2 inside the cabin is within -60 °C to about -15 °C and the flow velocity (*V*) of the fluidic stream(s) 201 is within a range of 1-15 m/s. Based on the measurements obtainable from the thermal sensor devices / arrays 13 in real time, also direction vectors for the fluidic stream(s) 201 can be adjusted by the blower devices 23 and optionally by the auxiliary fluid distribution devices 22.

In some instances, the cryocabin arrangement 100 is pre-cooled before the operating cycle to achieve the predetermined temperatures (*t*2) of the fluidic flow. Pre-cooling may take about 1-2 hours,
For clarity purposes the expression "cold-induced thermal sensory response" is utilized in the present disclosure with reference to processes occurring in skin and the underlying tissue during and instantly after the controlled cold impact (cold shock) and generally having beneficial effects on blood flow, cell and muscle metabolism and nerve conductance velocity. Controlled cold impact triggers for example vasomotor responses, such as cold-induced alteration of the diameter in peripheral blood vessels (hereby, narrowing or vasoconstriction and subsequent widening or vasodilatation) leading to acceleration and intensification of blood circulation and increased oxygen supply to blood and tissues; and associated release of neuromediator substances, such as noradrenaline, serotonin and endorphins, into bloodstream. Amongst aforesaid, noradrenaline in known for its involvement in reducing inflammation processes and serotonin and endorphins are often referred to as natural painkillers. Upon controlled cold impact, musculoskeletal inflammation and swelling are reduced thus relieving pain and spasm, in particular, around joints and tendons. Overall, a sufficiently intense cold-induced thermal sensory response promotes restorative processes in the body and generally imposes positive effects on the user's health and wellbeing.

The cryocabin arrangement 100 generally exploits a so-called "wind chill factor" principle (whereby the body feels far colder than the ambient temperature due to the "wind", viz. the flow of cold air), which together with the containment of a cooling medium stream maximizes heat extraction capacity from skin and subcutaneous tissue in all treated areas (in present case, over the entire body of the treated subject). The wind chill factor effect is attainable by directing cooling fluid adjusted to a predetermined temperature inside an enclosure (the cabin 10 optionally closed with a top cover 14) at a predetermined flow velocity, whereby a uniform high-speed flow is established essentially along the entire height of the cabin.

In the method, the temperature (*t2*) of the cooling fluid distributed inside the cabin 10 is adjusted to temperature values equal to or above -60 degree Celsius (°C), preferably, the temperature values within a range of between about -60 °C to about -15 °C. Flow velocity (*V*) of a stream of the cooling fluid distributed inside the cabin is adjusted to a range of between about 1-15 m/s.

The method may further comprise inputting into the data processing unit 30 supplementary user-specific data selected from the group consisting of: height, weight, age, and gender, before the beginning of the operating cycle, and optionally combining said supplementary user specific data with the user-specific temperature measurement data (*t1*) obtainable during the operation cycle.

Some aspects of the invention may be implemented in a computer program product i.e. a collection of computer program instructions stored on a computer readable storage device for execution by a computer. The computer program product provided hereby is adapted to perform the computer implemented method according to the embodiments. These instructions can be provided as complete executable programs, as modifications to existing programs or extensions ("plugins") for existing programs.

That highly uniform cooling can be achieved by the cryocabin arrangement 100 of the invention, is illustrated by the following non-limiting examples.

### Examples:

The examples provided herewith illustrate measurement results obtained in the electrically operated cryocabin arrangement 100 according to the embodiments (Cabin 2) as compared to whole-body cryocabin devices cooled with liquid nitrogen (Cabin 1 and Cabin 3). Cabin 1 was liquid nitrogen powered CTN CryoCabin (Cryotech Finland Oy), and Cabin 3 was a liquid nitrogen powered whole-body cabin device from a foreign manufacturer. All cabins were calibrated 4-6 days before the measurements.

Skin temperature profiles of twelve persons (8 males and 4 females) aged 24-71 were measured before and after a treatment in all tested cryocabins. In all measurements, the temperature scale was set to a range of 0-35 °C. Exemplary measurement results for two persons are provided herein below.

*Testing method:* Thermal images were taken from a distance of approximately 6 m. Location was chosen close to the cabinet to ensure short delay (about 10 seconds) between the treatment and the photographs. Two thermal images, front and rear side of the whole person, were taken before and after the treatment. The tests were carried out on three separate days to ensure similar starting conditions and comparable skin temperatures of the test objects. On each day, a different cabin was used.

*Measurement standards used in calibration and traceability:* Thermal images were taken with FLIR E54 24° thermal camera.

*Testing conditions:* Tests were performed under ordinary room conditions. Ambient temperature was approximately Tₐ = (22 ± 2) °. Relative humidity was not monitored.

*Measurements*: Liquid nitrogen operated Cabins 1 and 3 were set at a temperature of -130 °C, and the duration of the treatment was 180 seconds for all test subjects. Cabin 2 was cooled electrically. The cold distribution and duration were controlled automatically by the temperature monitoring system integrated in the device. Temperature of the cooling fluid (*t*2) was within a range of -35 °C to -20 °C. The treatment was continued until the chosen skin temperature of +5 °C was reached. The duration of the treatment varied from 109 seconds to a set maximum of 180 seconds depending on the person's individual characteristics.

Measurement analysis for the following two users is provided herein below.

User 1: Female, age 51, height 170 cm and weight 60 kg. User 2: Male, age 45, height 172 cm and wight 82 kg. Thermal images of subjects "User 1" and "User 2" were analyzed with a program FLIR Thermal Studio (Teledyne FLIR LLC). Eight measurement points were added to the images. Measurement point locations were Chest (location 1), Belly (location 2), Right arm (location 3), Left arm (location 4), Right thigh (location 5), Left thigh (location 6), Right leg (location 7), and Left leg (location 8). Temperatures were recorded and tabulated, and averages and standard deviations (Stdev) were calculated.

The results are summarized in Tables 1-4.

In Tables 1-4, all temperatures indications are given in °C.

**Table 1. Measured front side temperatures (t1) for the User 1.**

| Measurement Location | | Cabin 1 | | Cabin 2 (100) | | Cabin 3 | |
|---|---|---|---|---|---|---|---|
| | | Before | After | Before | After | Before | After |
| Chest | 1 | 33.9 | 21.8 | 34.3 | 14.2 | 33.7 | 24.1 |
| Belly | 2 | 33.0 | 16.4 | 33.0 | 9.2 | 32.1 | 19.8 |
| Right arm | 3 | 31.5 | 19.3 | 32.3 | 9.7 | 30.9 | 23.0 |
| Left arm | 4 | 31.0 | 19.3 | 31.6 | 13.8 | 30.4 | 25.8 |
| Right thigh | 5 | 30.3 | 5.3 | 30.6 | 12.3 | 28.8 | 7.3 |
| Left thigh | 6 | 28.7 | 8.7 | 30.8 | 11.2 | 28.7 | 10.7 |
| Right leg | 7 | 30.0 | 15.4 | 31.6 | 19.7 | 31.2 | 17.3 |
| Left leg | 8 | 31.3 | 19.3 | 32.4 | 18.0 | 31.7 | 18.5 |
| Average | | 31.4 | 15.1 | 32.1 | 13.5 | 30.9 | 18.3 |
| Stdev | | 1.5 | 5.4 | 1.1 | 3.5 | 1.6 | 6.1 |

**Table 2. Measured rear side temperatures (t1) for the User 1.**

| Measurement Location | | Cabin 1 | | Cabin 2 (100) | | Cabin 3 | |
|---|---|---|---|---|---|---|---|
| | | Before | After | Before | After | Before | After |
| Chest | 1 | 33.9 | 24.7 | 34.7 | 16.4 | 34.1 | 26.9 |
| Belly | 2 | 34.1 | 21.3 | 34.7 | 17.2 | 34.6 | 26.1 |
| Right arm | 3 | 31.2 | 13.6 | 32.4 | 12.6 | 32.4 | 19.0 |
| Left arm | 4 | 20.3 | 14.0 | 31.8 | 17.4 | 32.0 | 22.5 |
| Right thigh | 5 | 28.5 | 8.7 | 30.9 | 12.1 | 30.0 | 12.6 |
| Left thigh | 6 | 30.1 | 5.2 | 31.4 | 12.1 | 30.1 | 7.4 |
| Right leg | 7 | 28.3 | 9.5 | 30.8 | 15.8 | 28.9 | 12.0 |
| Left leg | 8 | 28.4 | 2.4 | 30.4 | 12.0 | 28.6 | 7.3 |
| Average | | 29.4 | 12.4 | 32.1 | 14.5 | 31.3 | 16.7 |
| Stdev | | 4.1 | 7.1 | 1.6 | 2.3 | 2.1 | 7.5 |

**Table 3. Measured front side temperatures (t1) for the User 2.**

| Measurement Location | | Cabin 2 | | Cabin 2 (100) | | Cabin 3 | |
|---|---|---|---|---|---|---|---|
| | | Before | After | Before | After | Before | After |
| Chest | 1 | 33.1 | 25.0 | 33.2 | 21.3 | 32.6 | 26.9 |
| Belly | 2 | 30.5 | 12.0 | 31.7 | 7.1 | 30.7 | 14.7 |
| Right arm | 3 | 32.0 | 25.4 | 33.2 | 18.4 | 31.4 | 24.3 |
| Left arm | 4 | 31.8 | 19.9 | 32.7 | 21.6 | 31.1 | 23.7 |
| Right thigh | 5 | 28.2 | 6.8 | 30.6 | 17.6 | 28.1 | 11.4 |
| Left thigh | 6 | 28.2 | 15.0 | 30.4 | 18.5 | 29.4 | 16.5 |
| Right leg | 7 | 32.8 | 20.1 | 33.2 | 23.5 | 32.1 | 23.1 |
| Left leg | 8 | 32.4 | 20.3 | 33.9 | 17.7 | 31.9 | 23.6 |
| Average | | 31.1 | 18.1 | 32.4 | 18.2 | 30.9 | 20.5 |
| Stdev | | 1.8 | 6.0 | 1.2 | 4.7 | 1.4 | 5.2 |

**Table 4. Measured rear side temperatures (t1) for the User 2.**

| Measurement Location | | Cabin 2 | | Cabin 2 (100) | | Cabin 3 | |
|---|---|---|---|---|---|---|---|
| | | Before | After | Before | After | Before | After |
| Chest | 1 | 32.9 | 20.5 | 33.3 | 6.6 | 32.5 | 25.7 |
| Belly | 2 | 32.9 | 23.7 | 32.8 | 16.1 | 32.9 | 27.1 |
| Right arm | 3 | 30.5 | 11.9 | 32.6 | 13.4 | 31.0 | 18.4 |
| Left arm | 4 | 31.6 | 16.9 | 32.5 | 14.9 | 30.6 | 18.9 |
| Right thigh | 5 | 30.4 | 14.1 | 31.7 | 15.5 | 30.3 | 16.4 |
| Left thigh | 6 | 30.4 | 7.2 | 31.4 | 15.4 | 30.2 | 10.4 |
| Right leg | 7 | 30.9 | 11.2 | 31.9 | 17.1 | 29.7 | 13.7 |
| Left leg | 8 | 30.9 | 0.2 | 31.9 | 16.1 | 30.1 | 10.8 |
| Average | | 31.3 | 13.2 | 32.3 | 14.4 | 30.9 | 17.7 |
| Stdev | | 1.0 | 7.0 | 0.6 | 3.1 | 1.1 | 5.8 |

It is clear to a person skilled in the art that with the advancement of technology the basic ideas of the present invention may be implemented in various ways. The invention and its embodiments may generally vary within the scope of the appended claims.

## Claims

1. An electrically operated cryocabin arrangement (100) comprising an open-top cabin (10) with a number of thermal sensor arrays (13) arranged at different heights along an internal perimeter of the cabin, a number of cooling units (20), and a data processing unit (30),
wherein the data processing unit is configured to receive user-specific data comprising at least temperature indications (*t1*) measureable at skin surface of a user by the thermal sensor arrays (13) throughout an operation cycle, during which cycle a cooling fluid (201) is delivered into the cabin and distributed inside the cabin via the cooling units (20), and
based on said user-specific data, to selectively adjust variables related to distribution of the cooling fluid (201) inside the cabin to a predetermined level, said variables being at least a flow velocity (*V*) of a cooling fluid stream and/or a temperature (*t*2) of the cooling fluid,
wherein the cooling fluid distribution variables are adjusted to reach the values, at which temperature indications (*t1*) measureable at the user skin surface are within a range of about 0 degree Celsius (°C) to about 20 °C,
wherein adjustment is performed in real time, continuously throughout the operation cycle, and
wherein the processing unit (30) is configured to terminate the operating cycle automatically when a desired temperature value (*t1*) measureable at the user skin surface has been achieved, wherein the temperature (t1) is achieved, when a predetermined stability determination threshold time is reached or exceeded, wherein different body areas or groups of body areas have their own stability determination threshold time.

2. The cryocabin arrangement of claim 1, wherein the temperature (*t*2) of the cooling fluid distributed inside the cabin (10) is adjusted to a temperature value equal to or above about -60 °C, preferably, to the temperature value within a range of between about -60 °C to about -15 °C.

3. The cryocabin arrangement of any one of claims 1 or 2, wherein the flow velocity (V) of the cooling fluid stream distributed inside the cabin is adjusted to a range of between about 1-15 m/s.

4. The cryocabin arrangement of any preceding claim, wherein the data processing unit (30) is further configured to combine the user-specific temperature measurement data (t1) obtainable continuously throughout the operation cycle with a supplementary user-specific data selected from the group consisting of: height, weight, age, and gender, and inputted into the processing unit before the beginning of the operating cycle.

5. The cryocabin arrangement of any preceding claim, wherein the cooling unit (20) comprises a heat exchanger device (21), such as a heat exchanger evaporator device, and at least one blower device (23) configured to deliver, distribute, and preferably recirculate the cooling fluid inside the cabin.

6. The cryocabin arrangement of any preceding claim, in which recirculation of the cooling fluid is enabled between the cooling units (20) and the cabin (10) in a substantially closed-loop cycle.

7. The cryocabin arrangement of any preceding claim, wherein the cooling fluid is air.

8. The cryocabin arrangement of any preceding claim, wherein the thermal sensor arrays (13) are infrared sensor arrays, such as thermopiles.

9. The cryocabin arrangement of any preceding claim, wherein the cabin (10) further comprises a removable top cover (14) with a neck collar portion.

10. A computer program product adapted to perform a method (500) for operating a cryocabin arrangement (100) comprising an open-top cabin (10) with a number of thermal sensor arrays (13) arranged at different heights along an internal perimeter of the cabin, a number of cooling units (20), and a data processing unit (30), the method comprises:
- receiving (501), into the data processing unit (30), user-specific data comprising at least temperature indications (*t1*) non-invasively measured from skin surface of a user by the thermal sensor arrays (13) in a contactless manner,
- processing (502) the user-specific measurement data in the data processing unit (30), and
- based on processed user-specific measurement data, generating (503), in the data processing unit (30), an output signal or a series of output signals configured, when transmitted to the cooling units (20), to selectively adjust variables related to distribution of a cooling fluid (201) inside the cabin to a predetermined level, said variables being at least a flow velocity (*V*) of a cooling fluid stream and/or a temperature (*t2*) of the cooling fluid,
wherein the output signal(s) generated by the data processing unit (30) is/are configured to selectively adjust cooling fluid distribution variables to reach the values, at which temperature indications (*t1*) measureable at the user skin surface are within a range of about 0 °C to about 20 °C,
wherein the method steps (501), (502) and (503) are performed in real time, continuously throughout an entire operation cycle, during which cycle the cooling fluid is delivered into the cabin via the cooling unit (20) and recirculated inside said cabin by fluid circulation units (21), and
wherein the processing unit (30) is configured to terminate the operating cycle automatically when a desired temperature value (*t1*) measureable at the user skin surface has been achieved, wherein the temperature (t1) is achieved, when a predetermined stability determination threshold time is reached or exceeded, wherein different body areas or groups of body areas have their own stability determination threshold time.

11. The computer program product of claim 10, in which the temperature (t2) of the cooling fluid distributed inside the cabin (10) is adjusted to a temperature value equal to or above about -60 °C, preferably, to the temperature value within a range of between about -60 °C to about -15 °C, and wherein the flow velocity (V) of the cooling fluid stream distributed inside the cabin is adjusted to a range of between about 1-15 m/s.

12. The computer program product of any one of claims 10 or 11, further comprising inputting into the data processing unit (30) supplementary user-specific data selected from the group consisting of: height, weight, age, and gender, before the beginning of the operating cycle, and optionally combining said supplementary user specific data with the user-specific temperature measurement data (t1) obtainable during the operation cycle.

## Patentansprüche

1. Elektrisch betriebene Kryokabinenanordnung (100), umfassend eine oben offene Kabine (10) mit einer Anzahl von Thermosensorarrays (13), die in unterschiedlichen Höhen entlang eines Innenumfangs der Kabine angeordnet sind, einer Anzahl von Kühleinheiten (20) und einer Datenverarbeitungseinheit (30),
wobei die Datenverarbeitungseinheit ausgestaltet ist, um benutzerspezifische Daten zu empfangen, umfassend mindestens Temperaturangaben (*t1*), die an der Hautoberfläche eines Benutzers durch die Thermosensorarrays (13) über einen gesamten Betriebszyklus messbar sind, wobei während des Zyklus mittels der Kühleinheiten (20) ein Kühlfluid (201) in die Kabine abgegeben und innerhalb der Kabine verteilt wird, und
basierend auf den benutzerspezifischen Daten selektiv Variablen, die mit der Verteilung des Kühlfluids (201) innerhalb der Kabine in Beziehung stehen, auf ein vorbestimmtes Niveau einzustellen, wobei die Variablen mindestens eine Strömungsgeschwindigkeit (*V*) eines Kühlfluidstroms und/oder eine Temperatur (*t*2) des Kühlfluids sind,
wobei die Kühlfluidverteilungsvariablen eingestellt werden, um die Werte zu erreichen, bei denen Temperaturangaben (*t1*), die an der Hautoberfläche des Benutzers messbar sind, innerhalb eines Bereichs von etwa 0 Grad Celsius (°C) bis etwa 20 °C liegen,
wobei die Einstellung kontinuierlich in Echtzeit über den gesamten Betriebszyklus durchgeführt wird, und
wobei die Verarbeitungseinheit (30) ausgestaltet ist, um den Betriebszyklus automatisch zu beenden, wenn ein gewünschter Temperaturwert (*t1*), der an der Hautoberfläche des Benutzers messbar ist, erreicht worden ist, wobei die Temperatur (*t1*) erreicht ist, wenn eine vorbestimmte Schwellenzeit zur Stabilitätsbestimmung erreicht oder überschritten worden ist, wobei unterschiedliche Körperbereiche oder Gruppen von Körperbereichen ihre eigene Schwellenzeit zur Stabilitätsbestimmung haben.

2. Kryokabinenanordnung nach Anspruch 1, wobei die Temperatur (*t2*) des innerhalb der Kabine (10) verteilten Kühlfluids auf einen Temperaturwert gleich oder oberhalb von etwa -60 °C, vorzugsweise auf einen Temperaturwert innerhalb eines Bereichs zwischen etwa -60 °C und etwa -15 °C eingestellt wird.

3. Kryokabinenanordnung nach einem der Ansprüche 1 oder 2, wobei die Strömungsgeschwindigkeit (V) des Kühlfluidstroms, der innerhalb der Kabine verteilt wird, auf einen Bereich zwischen etwa 1 und 15 m/s eingestellt wird.

4. Kryokabinenanordnung nach einem der vorhergehenden Ansprüche, wobei die Datenverarbeitungseinheit (30) ferner ausgestaltet ist, um die benutzerspezifischen Temperaturmessdaten (t1), die kontinuierlich über den gesamten Betriebszyklus erhältlich sind, mit ergänzenden benutzerspezifischen Daten zu kombinieren, die ausgewählt sind aus der Gruppe bestehend aus: Körpergröße, Gewicht, Alter und Geschlecht, und die vor Beginn des Betriebszyklus in die Verarbeitungseinheit eingegeben werden.

5. Kryokabinenanordnung nach einem der vorhergehenden Ansprüche, wobei die Kühleinheit (20) eine Wärmetauschervorrichtung (21), wie eine Wärmetauscherverdampfervorrichtung, und mindestens eine Gebläsevorrichtung (23) umfasst, die ausgestaltet ist, um das Kühlfluid innerhalb der Kabine abzugeben, zu verteilen und vorzugsweise umzuwälzen.

6. Kryokabinenanordnung nach einem der vorhergehenden Ansprüche, wobei Umwälzen des Kühlfluids zwischen den Kühleinheiten (20) und der Kabine (10) in einem im Wesentlichen geschlossenen Kreislauf ermöglicht wird.

7. Kryokabinenanordnung nach einem der vorhergehenden Ansprüche, wobei das Kühlfluid Luft ist.

8. Kryokabinenanordnung nach einem der vorhergehenden Ansprüche, wobei die Thermosensorarrays (13) Infrarotsensorarrays sind, wie Thermosäulen.

9. Kryokabinenanordnung nach einem der vorhergehenden Ansprüche, wobei die Kabine (10) des Weiteren eine entfernbare obere Abdeckung (14) mit einem Halskragenteil umfasst.

10. Computerprogrammprodukt, das eingerichtet ist, um ein Verfahren (500) zum Betreiben einer Kryokabinenanordnung (100) durchzuführen, die eine oben offene Kabine (10) mit einer Anzahl von Thermosensorarrays (13), die in unterschiedlichen Höhen entlang eines Innenumfangs der Kabine angeordnet sind, einer Anzahl von Kühleinheiten (20) und einer Datenverarbeitungseinheit (30) umfasst, wobei das Verfahren umfasst:
- Empfangen (501) von benutzerspezifischen Daten, umfassend mindestens Temperaturangaben (t1), die nicht-invasiv an der Hautoberfläche eines Benutzers durch die Thermosensorarrays (13) in kontaktloser Weise gemessen werden, an der Datenverarbeitungseinheit (30),
- Verarbeiten (502) der benutzerspezifischen Messdaten in der Datenverarbeitungseinheit (30), und
- basierend auf den verarbeiteten benutzerspezifischen Messdaten Generieren (503) eines Ausgabesignals oder einer Reihe von Ausgabesignalen in der Datenverarbeitungseinheit (30), die ausgestaltet sind, um, wenn sie an die Kühleinheiten (20) übertragen werden, Variablen, die mit der Verteilung des Kühlfluids (201) innerhalb der Kabine in Beziehung stehen, selektiv auf ein vorbestimmtes Niveau einzustellen, wobei die Variablen mindestens eine Strömungsgeschwindigkeit (*V*) eines Kühlfluidstroms und/oder eine Temperatur (*t*2) des Kühlfluids sind,
wobei das Ausgabesignal/die Ausgabesignale, das/die durch die Datenverarbeitungseinheit (30) generiert wird/werden, ausgestaltet ist/sind, um selektiv Kühlfluidverteilungsvariablen einzustellen, um die Werte zu erreichen, bei denen Temperaturangaben (*t1*), die an der Hautoberfläche des Benutzers messbar sind, innerhalb eines Bereichs von etwa 0 °C bis etwa 20 °C liegen,
wobei die Verfahrensschritte (501), (502) und (503) in Echtzeit kontinuierlich über einen gesamten Betriebszyklus durchgeführt werden, wobei während des Zyklus das Kühlfluid über die Kühleinheit (20) in die Kabine abgegeben wird und durch Fluidumwälzeinheiten (21) innerhalb der Kabine umgewälzt wird, und
wobei die Verarbeitungseinheit (30) ausgestaltet ist, um den Betriebszyklus automatisch zu beenden, wenn ein gewünschter Temperaturwert (*t1*), der an der Hautoberfläche des Benutzers messbar ist, erreicht worden ist, wobei die Temperatur (*t1*) erreicht ist, wenn eine vorbestimmte Schwellenzeit zur Stabilitätsbestimmung erreicht oder überschritten worden ist, wobei unterschiedliche Körperbereiche oder Gruppen von Körperbereichen ihre eigene Schwellenzeit zur Stabilitätsbestimmung haben.

11. Computerprogramm nach Anspruch 10, wobei die Temperatur (t2) des Kühlfluids, das innerhalb der Kabine (10) verteilt wird, auf einen Temperaturwert gleich oder oberhalb von etwa -60 °C, vorzugsweise auf den Temperaturwert innerhalb eines Bereichs zwischen etwa -60 °C und etwa -15 °C eingestellt wird, und wobei die Strömungsgeschwindigkeit (*V*) des Kühlfluidstroms, der innerhalb der Kabine verteilt wird, auf einen Bereich zwischen etwa 1 und 15 m/s eingestellt wird.

12. Computerprogrammprodukt nach einem der Ansprüche 10 oder 11, des Weiteren umfassend Eingeben von ergänzenden benutzerspezifischen Daten, ausgewählt aus der Gruppe bestehend aus: Körpergröße, Gewicht, Alter und Geschlecht, in die Datenverarbeitungseinheit (30) vor Beginn des Betriebszyklus, und gegebenenfalls Kombinieren der ergänzenden benutzerspezifischen Daten mit den benutzerspezifischen Temperaturmessdaten (*t1*), die während des Betriebszyklus erhältlich sind.

## Revendications

1. Dispositif de cabine cryogénique à commande électrique (100) comprenant une cabine à toit ouvert (10) avec un certain nombre de réseaux de capteurs thermiques (13) disposés à différentes hauteurs le long d'un périmètre interne de la cabine, un certain nombre d'unités de refroidissement (20) et une unité de traitement de données (30),
dans lequel l'unité de traitement de données est configurée pour recevoir des données spécifiques à l'utilisateur comprenant au moins des indications de température (*t1*) mesurables au niveau de la surface de la peau d'un utilisateur par les réseaux de capteurs thermiques (13) tout au long d'un cycle de fonctionnement, pendant lequel cycle un fluide de refroidissement (201) est acheminé dans la cabine et distribué à l'intérieur de la cabine par l'intermédiaire des unités de refroidissement (20), et sur la base desdites données spécifiques à l'utilisateur, pour ajuster de manière sélective des variables liées à la distribution du fluide de refroidissement (201) à l'intérieur de la cabine à un niveau prédéterminé, lesdites variables étant au moins une vélocité d'écoulement (V) d'un flux de fluide de refroidissement et/ou une température (*t2*) du fluide de refroidissement,
dans lequel les variables de distribution du fluide de refroidissement sont ajustées pour atteindre les valeurs auxquelles les indications de température (*t1*) mesurables au niveau de la surface de la peau de l'utilisateur se situent dans une plage comprise entre environ 0 degré Celsius (°C) et environ 20 °C,
dans lequel l'ajustement est effectué en temps réel, de manière continue tout au long du cycle de fonctionnement, et
dans lequel l'unité de traitement (30) est configurée pour mettre fin automatiquement au cycle de fonctionnement quand une valeur de température souhaitée (*t1*) mesurable au niveau de la surface de la peau de l'utilisateur a été atteinte, dans lequel la température (t1) est atteinte quand un temps seuil de détermination de stabilité prédéterminé est atteint ou dépassé,
dans lequel différentes zones du corps ou groupes de zones du corps ont leur propre temps seuil de détermination de stabilité.

2. Dispositif de cabine cryogénique selon la revendication 1, dans lequel la température (*t2*) du fluide de refroidissement distribué à l'intérieur de la cabine (10) est ajustée à une valeur de température supérieure ou égale à environ -60 °C, de préférence à une valeur de température dans une plage comprise entre environ - 60 °C et environ -15 °C.

3. Dispositif de cabine cryogénique selon l'une quelconque des revendications 1 ou 2, dans lequel la vélocité d'écoulement (V) du flux de fluide de refroidissement distribué à l'intérieur de la cabine est réglée dans une plage comprise entre environ 1 et 15 m/s.

4. Dispositif de cabine cryogénique selon l'une quelconque des revendications précédentes, dans lequel l'unité de traitement de données (30) est en outre configurée pour combiner les données de mesure de température spécifiques à l'utilisateur (t1) pouvant être obtenues en continu tout au long du cycle de fonctionnement avec des données supplémentaires spécifiques à l'utilisateur sélectionnées dans le groupe constitué par : la taille, le poids, l'âge et le sexe, et saisies dans l'unité de traitement avant le début du cycle de fonctionnement.

5. Dispositif de cabine cryogénique selon l'une quelconque des revendications précédentes, dans lequel l'unité de refroidissement (20) comprend un dispositif d'échange de chaleur (21), tel qu'un dispositif d'évaporation à échange de chaleur, et au moins un dispositif de ventilation (23) configuré pour fournir, distribuer et, de préférence, faire recirculer le fluide de refroidissement à l'intérieur de la cabine.

6. Dispositif de cabine cryogénique selon l'une quelconque des revendications précédentes, dans lequel la recirculation du fluide de refroidissement est activée entre les unités de refroidissement (20) et la cabine (10) dans un cycle en boucle fermée.

7. Dispositif de cabine cryogénique selon l'une quelconque des revendications précédentes, dans lequel le fluide de refroidissement est l'air.

8. Dispositif de cabine cryogénique selon l'une quelconque des revendications précédentes, dans lequel les réseaux de capteurs thermiques (13) sont des réseaux de capteurs infrarouges, tels que des thermopiles.

9. Dispositif de cabine cryogénique selon l'une quelconque des revendications précédentes, dans lequel la cabine (10) comprend en outre un couvercle supérieur amovible (14) avec une partie formant collier.

10. Produit de programme informatique adapté pour exécuter un procédé (500) destiné à faire fonctionner un dispositif de cabine cryogénique (100) comprenant une cabine à toit ouvert (10) avec un certain nombre de réseaux de capteurs thermiques (13) disposés à différentes hauteurs le long d'un périmètre interne de la cabine, un certain nombre d'unités de refroidissement (20) et une unité de traitement de données (30), le procédé comprenant :
- la réception (501), dans l'unité de traitement de données (30), de données spécifiques à l'utilisateur comprenant au moins des indications de température (*t1*) mesurées de manière non invasive à partir de la surface de la peau d'un utilisateur par les réseaux de capteurs thermiques (13) selon un mode sans contact,
- le traitement (502) des données de mesure spécifiques à l'utilisateur dans l'unité de traitement de données (30), et
- sur la base des données de mesure spécifiques à l'utilisateur traitées, la génération (503), dans l'unité de traitement de données (30), d'un signal de sortie ou d'une série de signaux de sortie configurés, quand ils sont transmis aux unités de refroidissement (20), pour ajuster de manière sélective des variables liées à la distribution d'un fluide de refroidissement (201) à l'intérieur de la cabine à un niveau prédéterminé, lesdites variables étant au moins une vélocité d'écoulement (V) d'un flux de fluide de refroidissement et/ ou une température (*t2*) du fluide de refroidissement,
dans lequel le ou les signaux de sortie générés par l'unité de traitement de données (30) sont configurés pour ajuster de manière sélective les variables de distribution du fluide de refroidissement afin d'atteindre les valeurs auxquelles les indications de température (*t1*) mesurables au niveau de la surface de la peau de l'utilisateur se situent dans une plage comprise entre environ 0 °C et environ 20 °C,
dans lequel les étapes de procédé (501), (502) et (503) sont exécutées en temps réel, de manière continue tout au long d'un cycle de fonctionnement complet, pendant lequel le fluide de refroidissement est acheminé dans la cabine par l'intermédiaire de l'unité de refroidissement (20) et recirculé à l'intérieur de ladite cabine par des unités de circulation de fluide (21), et
dans lequel l'unité de traitement (30) est configurée pour mettre fin automatiquement au cycle de fonctionnement quand une valeur de température souhaitée (*t1*) mesurable au niveau de la surface de la peau de l'utilisateur a été atteinte, dans lequel la température (t1) est atteinte quand un temps seuil de détermination de stabilité prédéterminé est atteint ou dépassé,
dans lequel différentes zones du corps ou groupes de zones du corps ont leur propre temps seuil de détermination de stabilité.

11. Produit de programme informatique selon la revendication 10, dans lequel la température (t2) du fluide de refroidissement distribué à l'intérieur de la cabine (10) est ajustée à une valeur de température supérieure ou égale à environ -60 °C, de préférence à une valeur de température dans une plage comprise entre environ -60 °C et environ -15 °C, et dans lequel la vélocité d'écoulement (V) du flux de fluide de refroidissement distribué à l'intérieur de la cabine est ajustée à une plage comprise entre environ 1 et 15 m/s.

12. Produit de programme informatique selon l'une quelconque des revendications 10 ou 11, comprenant en outre l'entrée dans l'unité de traitement de données (30) de données supplémentaires spécifiques à l'utilisateur sélectionnées dans le groupe constitué par : la taille, le poids, l'âge et le sexe, avant le début du cycle de fonctionnement, et combinant éventuellement lesdites données supplémentaires spécifiques à l'utilisateur avec les données de mesure de température spécifiques à l'utilisateur (t1) pouvant être obtenues pendant le cycle de fonctionnement.
